(19) Europäisches Patentamt / European Patent Office / Office européen des brevets

(11) **EP 4 531 132 A2**

(12) **EUROPEAN PATENT APPLICATION**


(43) Date of publication:
**02.04.2025 Bulletin 2025/14**

(21) Application number: **24202496.6**

(22) Date of filing: **25.09.2024**

(51) International Patent Classification (IPC):
***H01M 4/131*** *(2010.01)* ***H01M 4/1391*** *(2010.01)*
***H01M 4/525*** *(2010.01)* ***H01M 4/62*** *(2006.01)*
*H01M 4/02* *(2006.01)*

(52) Cooperative Patent Classification (CPC):
**H01M 4/131; H01M 4/1391; H01M 4/525; H01M 4/62; H01M 4/623;** H01M 2004/028

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.09.2023 CN 202311271845**



(71) Applicant: **Ningde Amperex Technology Ltd.**
**Ningde City, Fujian 352100 (CN)**

(72) Inventor: **ZHOU, Changtong**
**Ningde City, Fujian 352100 (CN)**

(74) Representative: **Icosa**
**83 avenue Denfert-Rochereau**
**75014 Paris (FR)**


(54) **POSITIVE ELECTRODE ADDITIVE, POSITIVE ELECTRODE PLATE AND PREPARATION METHOD THEREOF, AND APPLICATION THEREOF**


(57) A positive electrode additive includes a structure shown in Formula I. In Formula I, $R_1$ and $R_2$ are respectively independently selected from $C_2$-$C_{18}$ alkyls. When the positive electrode additive is applied to the preparation of a positive electrode plate of a lithium ion battery, the flexibility of the electrode plate can be significantly improved, the risk of brittle failure of the electrode plate in the winding process due to the hardness and brittleness can be avoided, and the P.D of the electrode plate can be increased, thereby increasing the energy density of the lithium ion battery.

Formula I

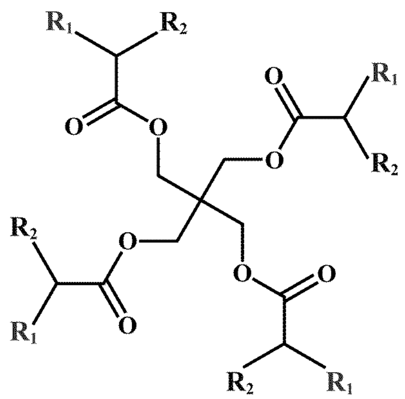





Processed by Luminess, 75001 PARIS (FR)

## Description

### TECHNICAL FIELD

This application relates to the technical field of batteries, and in particular to a positive electrode additive, a positive electrode plate and a preparation method thereof, and application thereof.

### BACKGROUND

Lithium ion batteries are widely applied in the field of 3C consumer products due to their long cycle life and high energy density. With the rapid development of a mobile electronic device, people have higher and higher requirements for the dynamics performance and long cycle life of the lithium ion batteries. Polyvinylidene fluoride (PVDF) is generally used as a positive electrode binder of a lithium ion battery. However, a positive electrode plate prepared by the PVDF binder is relatively hard and brittle, so there is a risk of brittle failure in the winding process. Meanwhile, the pellet density (P.D) of the obtained positive electrode plate is low, thereby seriously reducing the energy density of the lithium ion battery.

To avoid the risk of brittle failure of the positive electrode plate in the winding process due to the hardness and brittleness, residual N-methylpyrrolidone (NMP) in the positive electrode will be generally increased in the prior art to solve the problem of hardness and brittleness, while to obtain a higher energy density, the adding amount of non-active materials will be generally reduced in the prior art. However, excessive NMP remaining in the electrode plate will cause problems such as flatulence and abnormal thickness in a cell system, and reducing the adding amount of the non-active materials in the electrode plate will reduce the electronic conductivity of the electrode plate and the binding force between the active material and a substrate.

### SUMMARY

In view of this, this application provides a positive electrode additive, a positive electrode plate and a preparation method thereof, and application thereof. When the positive electrode additive is applied to the preparation of an electrode plate of a lithium ion battery, the flexibility of the electrode plate can be significantly improved, thereby increasing the pellet density (P.D) of the electrode plate.

According to a first aspect, this application provides a positive electrode additive. The positive electrode additive includes a structure shown in Formula I:

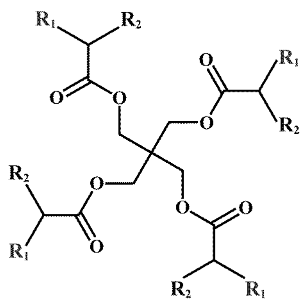

Formula I;

where in Formula I, $R_1$ and $R_2$ are respectively independently selected from $C_2$-$C_{18}$ alkyls.

In combination with Formula I, the positive electrode additive is a micromolecular additive containing a plurality of polar ester groups connected to branched-chain alkyl structures. A positive electrode plate prepared by the positive electrode additive with the shown structure has better flexibility, so that the risk of brittle failure of the positive electrode plate in the winding process due to the hardness and brittleness can be avoided, and the P.D can be increased.

In some embodiments, in Formula I, $R_1$ and $R_2$ are respectively independently selected from $C_{12}$-$C_{18}$ linear alkyls.

According to a second aspect, this application provides a positive electrode plate. The positive electrode plate includes a positive electrode current collector and a positive electrode active material layer arranged on at least one surface of the positive electrode current collector, where the positive electrode active material layer includes a positive electrode active material, a binder, a conducting agent and the positive electrode additive.

In some embodiments, based on the mass of the positive electrode active material layer, the mass percentage of the positive electrode additive is 0.05wt%-0.5wt%. Preferably, the mass percentage of the positive electrode additive is

0.3wt%-0.5wt%. In this application, the content of the positive electrode additive in the positive electrode active material layer is further regulated and controlled to be appropriate, so on one hand, it is beneficial to regulate and control the viscosity of positive electrode slurry to be appropriate, and on the other hand, the diaphragm resistance of the prepared positive electrode active material layer is low, and the cycle performance of a lithium ion battery is improved.

In some embodiments, the boiling point of the positive electrode additive is 300°C to 800°C. It can be seen that the positive electrode additive provided by this application has the characteristic of low volatility.

In some embodiments, the binder contains a C-F bond. Preferably, the binder includes polyvinylidene fluoride (PVDF). In the positive electrode additive provided by this application, a plurality of symmetrical polar ester groups facilitate the synergistic effect with the C-F bond in the PVDF and mutual dissolution; furthermore, the symmetrical structure is more beneficial to weakening the action force between PVDF molecular chains, improving the mobility of the PVDF molecular chains and reducing the glass transition temperature of the PVDF, thereby improving the flexibility of the electrode plate, solving the problem of hardness and brittleness of the electrode, and increasing P.D.

In some embodiments, the molecular weight of the binder is 800000 to 1200000. Preferably, the molecular weight of the PVDF is 1000000, which is more beneficial for the positive electrode additive to be inserted between the PVDF molecular chains, so that the non-polar long-chain alkyl of the positive electrode additive is clamped between the PVDF molecular chains, the polar C-F bond in the PVDF is shielded, and the action force between the PVDF molecular chains is reduced, thereby further improving the flexibility of the electrode plate; furthermore, the micromolecular additive (that is, the positive electrode additive) can absorb moisture in the air, and has the effects of lubrication and moisture retention, which facilitates the slip of main material particles during cold pressing, thereby reducing damage to an aluminum foil and further increasing P.D.

In some embodiments, the binding force between the positive electrode active material layer and the positive electrode current collector is 28.5 N/m to 29.5 N/m. It can be seen that the positive electrode additive provided by this application can improve the flexibility of the positive electrode plate without reducing the binding force between the positive electrode active material layer and the positive electrode current collector.

In some embodiments, the diaphragm resistance of the positive electrode plate is 0.2 Q to 0.25 Q. It can be seen that the positive electrode additive provided by this application can improve the electronic conductivity of the electrode plate while improving the flexibility of the positive electrode plate.

According to a third aspect, this application provides a preparation method for a positive electrode plate, including the following steps:

dispersing the positive electrode active material, the binder, the conductive agent and the positive electrode additive into a non-aqueous solution, and stirring and mixing to obtain positive electrode slurry, where the solid content of the positive electrode slurry is 65wt% to 75wt%, and the viscosity of the positive electrode slurry is 3900 mPa s to 6100 mPa s.

In some embodiments, based on the solid content of the positive electrode slurry, the mass ratio of the positive electrode active material to the binder to the conductive agent to the positive electrode additive is (95.5-97):(1-2):(1-2):(0.05-0.5).

According to a fourth aspect, this application provides an electrochemical apparatus, including the above positive electrode plate.

According to a fifith aspect, this application provides an electronic device, including the above electrochemical apparatus.

The technical solutions provided by some embodiments of this application at least have the following beneficial effects: when the positive electrode additive provided by this application is applied to the preparation of the positive electrode plate of the lithium ion battery, the flexibility of the electrode plate can be significantly improved. The plurality of symmetrical polar ester groups in the positive electrode additive facilitate the synergistic effect with the C-F bond in the PVDF and mutual dissolution, so that the action force between the molecular chains of the binder is weakened, the crystallinity of the PVDF is reduced, the mobility of the molecular chains is improved, the glass-transition temperature is reduced, the flexibility of the electrode plate is improved, and the problem of hardness and brittleness of the electrode plate is solved; the non-polar long-chain alkyl of the positive electrode additive is clamped between the PVDF molecular chains, and the polar C-F bond in the PVDF is shielded, so that the action force between the PVDF molecular chains is reduced and the flexibility of the electrode plate is improved; furthermore, the positive electrode additive can absorb moisture in the air, has the characteristics of lubrication and moisture retention, and facilitates the slip of main material particles in the cold pressing process, thereby reducing the damage to an aluminum foil and increasing the P.D of the electrode plate.

## DETAILED DESCRIPTION

To make the objectives, technical solutions, and advantages of this application clearer, this application is further described below in detail with reference to the accompanying drawings and embodiments. It should be understood that the specific embodiments described herein are only for explaining this application, rather than for limiting this application.

Positive electrode additive

The positive electrode additive includes a structure shown in Formula I:

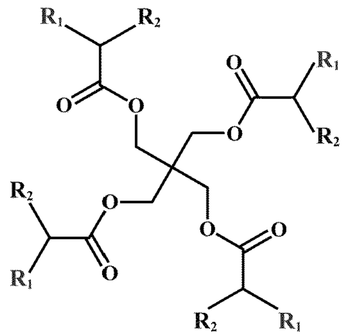

Formula I

In Formula I, $R_1$ and $R_2$ are respectively independently selected from $C_2$-$C_{18}$ alkyls.

In some embodiments, in Formula I, $R_1$ and $R_2$ are respectively independently selected from $C_{12}$-$C_{18}$ linear alkyls.

Preparation method for the positive electrode additive

Exemplarily:
pentaerythritol and branched-chain carboxylic acid compounds were mixed, a proper amount of sulfuric acid was added, a reaction was performed for 60-80 hours under mechanical stirring at 160°C-200°C and 250-350 rpm, a proper amount of drying agent was added after the reaction, and filtering was performed to obtain the positive electrode additive.

Positive electrode plate

The positive electrode plate includes a positive electrode current collector and a positive electrode active material layer arranged on at least one surface of the positive electrode current collector, where the positive electrode active material layer includes a positive electrode active material, a binder, a conducting agent and the above positive electrode additive.

In some embodiments, based on the mass of the positive electrode active material layer, the mass percentage of the positive electrode additive is 0.05wt%-0.5wt%. Preferably, the mass percentage of the positive electrode additive is 0.05wt%, 0.08wt%, 0. 1wt%, 0. 15wt%, 0.2wt%, 0.25wt%, 0.3wt%, 0.35wt%, 0.4wt%, 0.45wt%, 0.5wt% or in a range formed by any two of the above values. Preferably, the mass percentage of the positive electrode additive is 0.3wt%-0.5wt%.

In some embodiments, the molecular weight of the binder is 800000 to 1200000. Exemplarily, the molecular weight of the binder is 800000, 850000, 900000, 1000000, 1050000, 1100000, 1150000, 1200000 or in a range formed by any two of the above values.

In some embodiments, the binder contains a C-F bond. Preferably, the binder includes polyvinylidene fluoride.

In some embodiments, the positive electrode active material in the positive electrode active material layer may be selected from one or more of lithium cobalt oxide, lithium nickel oxide, lithium manganese oxide, lithium nickel manganese oxide, lithium nickel cobalt manganese oxide, lithium nickel cobalt aluminum oxide, lithium iron phosphate, and compounds obtained by adding other transition metals or nontransition metals into the above compounds.

In some embodiments, the positive electrode current collector may use a metal foil material or a porous metal plate, for example, use a foil material or porous plate of aluminum, copper, nickel, titanium or iron or alloy thereof, such as an aluminum (Al) foil.

In some embodiments, the binding force between the positive electrode active material layer and the positive electrode current collector is 28.5 N/m to 29.5 N/m. Exemplarily, the binding force between the positive electrode active material layer and the positive electrode current collector is 28.5 N/m, 28.8 N/m, 29 N/m, 29.2 N/m, 29.3 N/m, 29.5 N/m or in a range formed by any two of the above values.

In some embodiments, the diaphragm resistance of the positive electrode plate is 0.2 Q to 0.25 Q. Exemplarily, the diaphragm resistance of the positive electrode plate is 0.2 S2, 0.22 S2, 0.23 Q, 0.25 Q or in a range formed by any two of the above values.

Preparation method for the positive electrode plate

The preparation method for the positive electrode plate at least includes the following steps: the positive electrode active material, the binder, the conductive agent and the positive electrode additive were dispersed into a non-aqueous solution, and stirring and mixing were performed to obtain positive electrode slurry, where the solid content of the positive electrode slurry is 65wt% to 75wt%, the viscosity of the positive electrode slurry is 3900 mPa s to 6100 mPa·s, and based on the solid content of the positive electrode slurry, the mass ratio of the positive electrode active material to the binder to the conductive agent to the positive electrode additive is (95.5-97):(1-2):(1-2):(0.05-0.5).

One surface of a positive electrode current collector was coated with the positive electrode slurry uniformly, drying was performed to obtain a positive electrode plate with one surface coated with the positive electrode active material, and then another surface of the positive electrode current collector was subjected to the above operation steps to obtain a positive electrode plate with double surfaces coated with the positive electrode active material.

Exemplarily, the viscosity of the positive electrode slurry is 3900 mPa.s, 3950 mPa.s, 4000 mPa.s, 4500 mPa.s, 5000 mPa.s, 5500 mPa.s, 6000 mPa.s, 6100 mPa.s or in a range formed by any two of the above values.

Other

A negative electrode plate may be a lithium metal sheet, or may include a negative electrode current collector and a negative electrode active material layer arranged on at least one surface of the negative electrode current collector.

The negative electrode active material layer generally includes a negative electrode active material, an optional conductive agent and an optional binder.

Exemplarily, the negative electrode active material includes one or more of natural graphite, artificial graphite, a mesocarbon microbead (MCMB), hard carbon, silicon, a silicon-carbon compound, SiO, Li-Sn alloy, Li-Sn-O alloy, Li-Al alloy or lithium; the conductive agent includes one or more of conductive carbon, acetylene black, carbon black, Ketjen black, a carbon dot, a carbon nanotube, graphene or a carbon nanofiber; and the binder includes one or more of styrene butadiene rubber (SBR), polyvinylidene fluoride (PVDF), polytetrafluoroethylene (PTFE), polyvinyl butyral (PVB), a water-based acrylic resin or carboxymethylcellulose (CMC). However, this application is not limited to these materials, and may also use other materials that can be used as the negative electrode active material, the conductive agent and the binder of the lithium ion battery.

Exemplarily, the negative electrode current collector may use a metal foil material or a porous metal plate, for example, use a foil material or porous plate of copper, nickel, titanium, iron or alloy thereof, such as a copper foil.

The negative electrode plate may be prepared according to a conventional method in this field. Exemplarily, the negative electrode active material, the optional conductive agent and the optional binder were dispersed into a solvent to form uniform negative electrode slurry, a negative electrode current collector is coated with the negative electrode slurry, and the negative electrode plate was obtained through drying and cold-pressing processes, where the solvent may be N-methylpyrrolidone (NMP) or deionized water.

The separator film is not specifically limited herein, and may select any well-known porous structure separator films with electrochemical stability and chemical stability, for example, a single-layer or multi-layer thin film of one or more of a glass fiber, a nonwoven fabric, polyethylene (PE), polypropylene (PP) and polyvinylidene fluoride (PVDF).

Electrolyte includes an organic solvent, an electrolyte lithium salt and an additive. The type of the electrolyte may not be specifically limited by this application, and may be selected according to actual requirements.

Exemplarily, the organic solvent includes one or more of ethylene carbonate (EC), propylene carbonate (PC), methyl ethyl carbonate (EMC), diethyl carbonate (DEC), dimethyl carbonate (DMC), dipropyl carbonate (DPC), methyl propyl carbonate (MPC), ethyl propyl carbonate (EPC), butylene carbonate (BC), fluoroethylene carbonate (FEC), methyl formate (MF), methyl acetate (MA), ethyl acetate (EA), propyl acetate (PA), methyl propionate (MP), ethyl propionate (EP), propyl propionate (PP), methyl butyrate (MB), ethyl butyrate (EB), gamma-butyrolactone (GBL), sulfolane (SF), methylsulfonylmethane (MSM), ethyl methylsulfone (EMS) or diethyl sulfone (ESE), preferably two or more.

Exemplarily, the electrolyte lithium salt includes one or more of $LiPF_6$ (lithium hexafluorophosphate), $LiBF_4$ (lithium tetrafluoroborate), $LiClO_4$ (lithium perchlorate), LiFSI (lithium bis(fluorosulfonyl)imide), LiTFSI (lithium bis(trifluoromethanesulphonyl)imide), LiTFS (lithium trifluoromethanesulfonate), LiDFOB (lithium difluoro(oxalato)borate), LiBOB (lithium bis(oxalate)borate), $LiPO_2F_2$ (lithium difluorophosphate), LiDFOP (lithium difluorobis(oxalato)phosphate) or LiTFOP (lithium tetrafluoro(oxalato)phosphate).

The electrolyte may further optionally include other additives which may be any additive capable of being used as a lithium ion secondary battery. The additive is not specifically limited by this application and may be selected according to actual requirements. As an example, the additive may be one or more of vinylene carbonate (VC), vinylethylene carbonate (VEC), succinonitrile (SN), adiponitrile (AND), prop-1-ene-1,3-sultone (PST), tris(trimethylsilyl)phosphate (TMSP) or tris(trimethylsilyl) borate (TMSB).

Electrochemical apparatus

The electrochemical apparatus provided by this application may include any apparatus in which an electrochemical reaction occurs. The specific examples of the electrochemical apparatus include all types of primary batteries or secondary batteries. In particular, the electrochemical apparatus is a lithium secondary battery, including a lithium metal secondary battery, a lithium ion secondary battery, a lithium polymer secondary battery or a lithium ion polymer secondary battery.

Electronic device

The electronic device provided by this application includes any electrochemical apparatus provided by this application. The electronic device provided by this application may be applied to, but not limited to, a notebook computer, a pen input computer, a mobile computer, an e-book player, a portable phone, a portable fax machine, a portable copying machine, a portable printer, a stereo headset, a video cassette recorder, a liquid crystal television, a handheld cleaner, a portable CD machine, a mini disk, a transceiver, an electronic notebook, a calculator, a memory card, a portable recorder, a radio, a backup power supply, a motor, an automobile, a motorcycle, a power-assisted bicycle, a bicycle, a luminaire, a toy, a game machine, a clock, an electric tool, a flashlight, a camera, a large battery for household use, a lithium ion capacitor and the like.

The embodiments of this application are described in detail below with reference to embodiments and comparative examples. Unless otherwise stated, the parts, percentages and ratios listed below are all based on weight, and the raw materials used may be obtained through commercial purchase or through synthesis according to a conventional method.

Embodiment 1-1

Preparation of positive electrode additive

1 mol of pentaerythritol and 4 mol of 2-Ethylbutyric acid were added into a 500-mL flask, 0.1 mol of ammonium sulfate was added, a reaction was performed for 72 hours under mechanical stirring at 180°C and 300 rpm, 5 mol of anhydrous $CaCl_2$ was added after the reaction, and filtering was performed by a 200-nm filter film to obtain the positive electrode additive.

Preparation of positive electrode plate

$LiCoO_2$ (positive electrode active material), a PVDF binder (with the molecular weight of 100 w), conductive carbon black (conductive agent) and a positive electrode additive (shown in Table 1) were dispersed in a solvent NMP, and uniform stirring and mixing was performed to obtain positive electrode slurry with the solid content of 70 wt% and the viscosity of 3960 mPa·s, where the mass ratio of the $LiCoO_2$ to the PVDF to the conductive carbon black to the positive electrode additive in the solid components is 95.9:2:2:0.1 (the mass percentage sum of the positive electrode active material and the positive electrode additive is 96%). One surface of a 9-μm positive electrode current collector was coated with the positive electrode slurry uniformly, and drying was performed at 120°C to obtain a positive electrode plate with a single surface coated with the positive electrode active material, where the thickness of the positive electrode active material layer is 75 μm. Then, another surface of the positive electrode current collector aluminum foil was subjected to the above operation steps to obtain a positive electrode plate with double surfaces coated with the positive electrode active material. The coated positive electrode plate was subjected to cold pressing, and then was cut into sheet materials with the specification of 70mm×800mm for later use.

Preparation of negative electrode plate

Negative electrode active materials graphite, styrene butadiene rubber and sodium carboxymethyl cellulose were mixed in the mass ratio of 97.6:1.1:1.3, then deionized water was added as a solvent, and uniform stirring was performed to obtain negative electrode slurry with the solid content of 70wt%. One surface of a negative electrode current collector copper foil with the thickness of 6 μm was coated with the negative electrode slurry uniformly, drying was performed at 120°C to obtain a negative electrode plate with a single surface coated with a negative electrode active material layer with the thickness of 110 μm, and then another surface of the negative electrode current collector copper foil was subjected to the above operation steps to obtain a negative electrode plate with double surfaces coated with the negative electrode active material layer. The coated negative electrode plate was subjected to cold pressing, and then was cut into sheet materials with the specification of 74mm×800mm for later use.

Preparation of electrolyte

In a glove box inflated with a dry argon atmosphere, organic solvents propylene carbonate (PC), diethyl carbonate (DEC) and ethylene carbonate (EC) were mixed in the mass ratio of 1:1:1, and then lithium hexafluorophosphate ($LiPF_6$) was added into the mixed organic solvent for dissolving and uniform mixing to obtain electrolyte with the $LiPF_6$ concentration of 1.15 mol/L.

Preparation of separator film

A porous polyethylene thin film with the thickness of 16 μm was used as the separator film.

Preparation of lithium ion battery

The positive electrode plate, separator film and negative electrode plate prepared above were stacked sequentially and wound to obtain an electrode assembly. After tabs were welded, the electrode assembly was put into an aluminum-plastic film, drying was performed for 12 hours in a vacuum oven at 80°C for dehydration, the electrolyte prepared above was injected, and the lithium ion battery was obtained through processes of vacuum packaging, standing, formation (charging to 3.5 V under the constant current of 0.02 C and then charging to 3.9 V under the constant current of 0.1 C, capacity, shaping and the like. In this application, unless otherwise specified, preparation can be performed according to conventional technical means in this field.

Test method:

(1) Viscosity test

The viscosity of the positive electrode slurry was tested by a digital rotary viscometer (Shanghai Jingtian Electronic Instrument Co., Ltd., LVDV-1), and an appropriate rotor and an appropriate rotating speed were selected according to the measured slurry. The rotor was slowly inserted into the slurry for infiltration, the rotary viscometer was started when the liquid level of the slurry was in the middle of a rotor groove, and after two minutes, an indicator was read when the indicator remained unchanged, denoted as the viscosity of the prepared positive electrode slurry, with the unit of mPa·s.

(2) Binding force test

The cold-pressed positive electrode plate was stamped by a die to obtain a test sample strip with the length of 100 mm and the width of 20 mm. A surface of a steel plate was wiped up with alcohol, and a double-faced adhesive tape with the length of 55-70 mm and the width of 20 mm (NITTO.NO5000NS) was bonded on the steel plate without bubbles. The test sample strip was centrally bonded on the double-faced adhesive tape, with a test face downward. A paper tape with the length of 50-75 mm and the width equal to that of the test sample strip and one end of the test sample strip were connected and fixed with a crepe tape (high-adhesion masking paper), and a rubber press roll with the mass of 2 kg was pushed with hands to roll back and forth on the test sample strip for four times to obtain a test sample. The sample was tested by a tensile machine (Suns, Instron 3365). The test sample was fixed on a test platform, the paper tape was folded up by 90° and was fixed by a clamp, and then the paper tape was slowly pulled by the tensile machine at the speed of 10 mm/min until the positive electrode active material layer on the surface of the double-faced adhesive tape was separated from the positive electrode current collector, and the test was finished. The average tensile force value in a stable region was recorded as a binding force between the positive electrode active material layer and the positive electrode current collector, with the unit of N/m.

(3) P.D calculation

A cell electrode plate after capacity was die-cut into 12 small wafers (with the area of 1540.25$mm^2$) by a stamping knife die, the die-cut small wafers were put on a weighing table with tweezers after an electronic scale was cleared, the weights $m_1$, $m_2$, $m_3$, $m_4$, $m_5$, $m_6$, $m_7$, $m_8$, $m_9$, $m_{10}$, $m_{11}$ and$m_{12}$ of the small wafers were recorded. The thicknesses $h_{11}$, $h_{12}$, $h_{13}$ and $h_{14}$; $h_{21}$, $h_{22}$, $h_{23}$ and $h_{24}$; $h_{31}$, $h_{32}$, $h_{33}$ and $h_{34}$; $h_{41}$, $h_{42}$, $h_{43}$ and $h_{44}$; $h_{51}$, $h_{52}$, $h_{53}$ and $h_{54}$; $h_{61}$, $h_{62}$, $h_{63}$ and$h_{64}$; $h_{71}$, $h_{72}$, $h_{73}$ and $h_{74}$; $h_{81}$, $h_{82}$, $h_{83}$ and $h_{84}$; $h_{91}$, $h_{92}$, $h_{93}$ and $h_{94}$; $h_{101}$, $h_{102}$, $h_{103}$ and $h_{104}$; $h_{111}$, $h_{112}$, $h_{113}$ and$h_{114}$; and $h_{121}$, $h_{122}$, $h_{123}$ and $h_{124}$ of each small wafer at the upper, lower, left and right positions were measured by a ten-thousandth micrometer. The average values $h_1$, $h_2$, $h_3$, $h_4$, $h_5$, $h_6$, $h_7$, $h_8$, $h_9$, $h_{10}$, $h_{11}$ and $h_{12}$ of four thicknesses of each small wafers

were taken, the $P.D_1 = \frac{m_1 - m_{substrate}}{(h_1 - h_{substrate}) * 1540.25} * 1000$ , $P.D_2 = \frac{m_2 - m_{substrate}}{(h_2 - h_{substrate}) * 1540.25} * 1000$ , $P.D_3 = \frac{m_3 - m_{substrate}}{(h_3 - h_{substrate}) * 1540.25} * 1000$ , $P.D_4 = \frac{m_4 - m_{substrate}}{(h_4 - h_{substrate}) * 1540.25} * 1000$ , $P.D_5 = \frac{m_2 - m_{substrate}}{(h_2 - h_{substrate}) * 1540.25} * 1000$ , $P.D_6 = \frac{m_6 - m_{substrate}}{(h_6 - h_{substrate}) * 1540.25} * 1000$ , $P.D_7 = \frac{m_7 - m_{substrate}}{(h_7 - h_{substrate}) * 1540.25} * 1000$ , $P.D_8 = \frac{m_8 - m_{substrate}}{(h_8 - h_{substrate}) * 1540.25} * 1000$ , $P.D_9 = \frac{m_9 - m_{substrate}}{(h_9 - h_{substrate}) * 1540.25} * 1000$ , $P.D_{10} = \frac{m_{10} - m_{substrate}}{(h_4 - h_{substrate}) * 1540.25} * 1000$ , $P.D_{11} = \frac{m_{11} - m_{substrate}}{(h_{11} - h_{substrate}) * 1540.25} * 1000$ and $P.D_{12} = \frac{m_{12} - m_{substrate}}{(h_{12} - h_{substrate}) * 1540.25} * 1000$ of the small wafers were calculated through formulas, and the average values of the P.D of 12 small wafers were taken and recorded as the P.D of each embodiment or comparative example.

(4) Diaphragm resistance test

The diaphragm resistance of the cold-pressed electrode plate was tested by an electrode plate resistance instrument (BER2500) of Inital Energy Science&Technology, resistance reset and pressure reset were performed before use, the electrode plate was put under a probe for test, and the average value of the resistance values measured at 12 different position was taken and recorded as the diaphragm resistance value.

(5) Cycle performance test

The cycle performance was evaluated through the capacity retention ratio of the lithium ion battery. The lithium ion battery after formation was put in a constant-temperature environment of 25°C, the lithium ion battery was charged to 4.5 V with the constant current of 0.6 C and then was charged to the current of 0.05 C with a constant voltage, after 3 minutes after fully charged, the lithium ion battery was discharged to 3.0 V with 0.5 C, and the discharge capacity was recorded as $D_0$. Cycle test was performed according to the 0.6C charging/0.5C discharging process, with 500 circles, and the discharge capacity after the $500^{th}$ cycles was recorded as Di, where the capacity retention ratio (%) of the lithium ion battery after 500 cycles at normal temperature (25°C) is equal to $D_1$/D0×100%.

Embodiments 1-2 to Embodiments 1-19

Different from Embodiment 1-1, some parameters of the positive electrode plate in the preparation process are adjusted, as shown in Table 1, and the others are the same as Embodiment 1-1. It should be noted that in Embodiments 1-2 to Embodiments 1-18, the non-polar long-chain alkyls are non-polar linear chain alkyls, and at least one branched chain of the non-polar long-chain alkyl in Embodiment 1-19 is an irregular chain alkyl. Specifically, the structural formula of the positive electrode additive in Embodiment 1-19 is as follows:

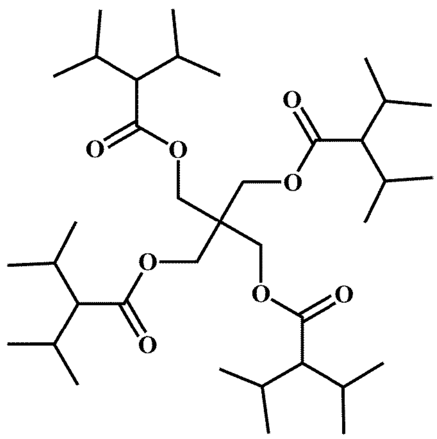

Comparative Example 1

Different from Embodiment 1-1, an additive is not used in the preparation process of the positive electrode slurry, the mass percentage content of the positive electrode active material is 96%, and the other is the same as Embodiment 1-1.

Comparative Example 2

Different from Embodiment 1-1, an additive used in the preparation process of the positive electrode slurry is succinic acid 1-ethyl 4-methyl ester, and the other is the same as Examples 1-1.

Table 1

| | The number of $R_1$ carbon atoms | The number of $R_2$ carbon atoms | Boiling point (°C) | The mass percentage content of additive (%) | Viscosity (mPa·s) |
|---|---|---|---|---|---|
| Embodiment 1-1 | 2 | 2 | 300 | 0.1 | 3960 |
| Embodiment 1-2 | 4 | 2 | 368 | 0.1 | 4023 |
| Embodiment 1-3 | 6 | 4 | 406 | 0.1 | 4325 |
| Embodiment 1-4 | 8 | 4 | 560 | 0.1 | 4678 |
| Embodiment 1-5 | 10 | 6 | 625 | 0.1 | 4897 |
| Embodiment 1-6 | 12 | 8 | 658 | 0.1 | 5032 |
| Embodiment 1-7 | 14 | 12 | 706 | 0.1 | 5325 |
| Embodiment 1-8 | 16 | 14 | 759 | 0.1 | 5568 |
| Embodiment 1-9 | 18 | 18 | 800 | 0.1 | 5860 |
| Embodiment 1-10 | 10 | 6 | 625 | 0.05 | 4043 |
| Embodiment 1-11 | 10 | 6 | 625 | 0.15 | 5015 |
| Embodiment 1-12 | 10 | 6 | 625 | 0.2 | 5368 |
| Embodiment 1-13 | 10 | 6 | 625 | 0.25 | 5432 |
| Embodiment 1-14 | 10 | 6 | 625 | 0.3 | 5578 |
| Embodiment 1-15 | 10 | 6 | 625 | 0.35 | 5690 |
| Embodiment 1-16 | 10 | 6 | 625 | 0.4 | 5870 |
| Embodiment 1-17 | 10 | 6 | 625 | 0.45 | 5930 |
| Embodiment 1-18 | 10 | 6 | 625 | 0.5 | 6056 |
| Embodiment 1-19 | 3 | 3 | 335 | 0.5 | 5787 |

Table 2

| | Diaphrag m Resistance (Ω) | Binding force (N/m) | P.D after capacity | Capacity retention ratio of 500 cycles (%) |
|---|---|---|---|---|
| Embodiment 1-1 | 0.25 | 29.2 | 3.86 | 87.2 |
| Embodiment 1-2 | 0.24 | 28.9 | 3.87 | 87.5 |
| Embodiment 1-3 | 0.24 | 28.9 | 3.88 | 87.3 |
| Embodiment 1-4 | 0.23 | 29.1 | 3.90 | 87.0 |
| Embodiment 1-5 | 0.22 | 28.9 | 3.92 | 87.1 |
| Embodiment 1-6 | 0.22 | 28.8 | 3.93 | 86.9 |
| Embodiment 1-7 | 0.21 | 29.0 | 3.95 | 86.8 |

(continued)

| | Diaphrag m Resistance (Ω) | Binding force (N/m) | P.D after capacity | Capacity retention ratio of 500 cycles (%) |
|---|---|---|---|---|
| Embodiment 1-8 | 0.22 | 28.8 | 3.97 | 87.2 |
| Embodiment 1-9 | 0.23 | 28.9 | 4.00 | 86.9 |
| Embodiment 1-10 | 0.24 | 29.2 | 3.88 | 87.0 |
| Embodiment 1-11 | 0.23 | 28.9 | 3.93 | 86.8 |
| Embodiment 1-12 | 0.22 | 29.1 | 3.94 | 87.5 |
| Embodiment 1-13 | 0.22 | 28.8 | 3.95 | 87.3 |
| Embodiment 1-14 | 0.22 | 28.9 | 3.96 | 87.0 |
| Embodiment 1-15 | 0.22 | 29.2 | 3.98 | 86.9 |
| Embodiment 1-16 | 0.21 | 28.7 | 3.98 | 86.9 |
| Embodiment 1-17 | 0.22 | 29.1 | 3.99 | 86.8 |
| Embodiment 1-18 | 0.23 | 29.0 | 4.00 | 87.2 |
| Embodiment 1-19 | 0.25 | 28.1 | 3.82 | 86.5 |
| Comparative Example 1 | 0.26 | 28.3 | 3.72 | 86.4 |
| Comparative Example 2 | 0.27 | 28.0 | 3.80 | 86.3 |

Referring to Table 1 and Table 2, it can be seen from the comparison between Embodiment 1-1 to Embodiment 1-18 and Comparative Example 1 that when the positive electrode plate of the lithium ion battery in the embodiments of this application contains the positive electrode additive provided by this application, the diaphragm resistance of the positive electrode plate, the binding force between the positive electrode active material layer and the current collector, and the capacity retention ratio of 500 cycles are improved compared with Comparative Example 1. Meanwhile, the micromolecular additive inserted between the PVDF molecular chains (that is, the positive electrode additive provided by this application) can weaken the action force between the PVDF molecular chains, improve the mobility of the molecular chains, the problem of hardness and brittleness of the electrode plate can be solved, the flexibility of the electrode plate can be improved, the P.D of the electrode plate after capacity can be improved significantly, the maximum P.D may be 4.00 g/cc, and the energy density of the lithium battery can be improved significantly.

The above are only preferred embodiments of this application and are not intended to limit this application. Any modifications, equivalent substitutions, improvements and the like made within the spirit and principles of this application should fall within the protection scope of this application.

## Claims

**1.** A positive electrode additive, comprising a structure shown in Formula I:

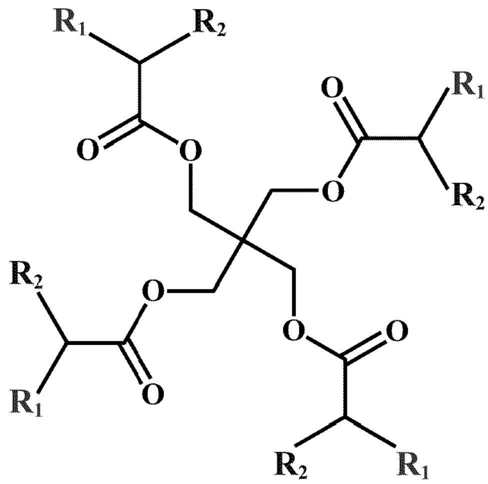

Formula I

$R_1$ and $R_2$ being respectively independently selected from $C_2$-$C_{18}$ alkyls.

2. The positive electrode additive according to claim 1, **characterized in that** $R_1$ and $R_2$ are respectively independently selected from $C_{12}$-$C_{18}$ linear alkyls.

3. A positive electrode plate, **characterized in that** the positive electrode plate comprises a positive electrode current collector and a positive electrode active material layer arranged on at least one surface of the positive electrode current collector,
the positive electrode active material layer comprises a positive electrode active material, a binder, a conductive agent and the positive electrode additive according to any one of claims 1 to 2.

4. The positive electrode plate according to claim 3, **characterized in that** based on a mass of the positive electrode active material layer, a mass percentage of the positive electrode additive is 0.05wt%-0.5wt%.

5. The positive electrode plate according to claim 4, **characterized in that** based on the mass of the positive electrode active material layer, the mass percentage of the positive electrode additive is 0.3wt%-0.5wt%.

6. The positive electrode plate according to claim 3, **characterized in that** a molecular weight of the binder is 800000 to 1200000.

7. The positive electrode plate according to claim 6, **characterized in that** the binder contains a C-F bond.

8. The positive electrode plate according to claim 3, **characterized in that** the binder comprises polyvinylidene fluoride.

9. A preparation method for the positive electrode plate according to any one of claims 3 to 8, the preparation method comprising the following steps:
dispersing the positive electrode active material, the binder, the conductive agent and the positive electrode additive into a non-aqueous solution, and stirring and mixing to obtain a positive electrode slurry, a solid content of the positive electrode slurry being 65wt% to 75wt%, and the viscosity of the positive electrode slurry being 3900 mPa·s to 6100 mPa·s.

10. The preparation method according to claim 9, **characterized in that** based on the solid content of the positive electrode slurry, the mass ratio of the positive electrode active material to the binder to the conductive agent to the positive electrode additive is (95.5-97):(1-2):(1-2):(0.05-0.5).

11. The positive electrode additive according to claim 1, **characterized in that** the boiling point of the positive electrode additive is 300°C to 800°C.

12. The positive electrode plate according to claim 3, **characterized in that** the binding force between the positive

electrode active material layer and the positive electrode current collector is 28.5 N/m to 29.5 N/m.

13. The positive electrode plate according to claim 3, **characterized in that** the diaphragm resistance of the positive electrode plate is 0.2 Q to 0.25 Q.

14. An electrochemical apparatus, comprising the positive electrode plate according to any one of claims 3 to 8.

15. An electronic device, comprising the electrochemical apparatus according to claim 14.